# NEW EUROPEAN PATENT SPECIFICATION

(11) **EP 2 123 270 B2**
(45) Date of publication and mention of the opposition decision: **15.06.2016**
(45) Mention of the grant of the patent: 29.08.2012
(21) Application number: 07850340.6
(22) Date of filing: 10.12.2007
(51) Int. Cl.: A61K 31/194, A61P 3/02, A61P 7/06, A61P 7/08, A61P 13/12, A61P 43/00, A61K 9/08, A61K 47/02, A61K 47/26

(54) **IRON METABOLISM-IMPROVING AGENT**
MITTEL ZUR VERBESSERUNG DES EISENSTOFFWECHSELS
AGENT AMÉLIORANT LE MÉTABOLISME DU FER

(30) Priority: 12.12.2006 JP 2006334294; 26.03.2007 JP 2007079488
(43) Date of publication of application: 25.11.2009
(73) Proprietor: Advanced Renal Technologies, Inc., Bellevue, WA 98005 (US)
(72) Inventor: NAKANISHI, Takeshi, Nishinomiya-shi Hyogo 663-8501 (JP); KURAGANO, Takahiro, Nishinomiya-shi Hyogo 663-8501 (JP); AKAIKE, Nobuhide, Shizuoka-shi Shizuoka 424-0911 (JP); NAKANISHI, Takashi, Chuo-ku Tokyo 104-8315 (JP)
(74) Representative: Nicholls, Kathryn Margaret
(86) International application number: PCT/JP2007/073766
(87) International publication number: WO 2008/072591

(56) References cited:
- EP-A1- 2 123 270
- WO-A1-01//00204
- WO-A1-98//06482
- WO-A1-2005/094918
- WO-A1-2006/073164
- WO-A1-2006/130065
- WO-A1-2005//002599
- JP-A- 10 087 478
- JP-A- 2003 104 869
- US-A1- 2002 004 530
- US-B1- 6 779 468
- YUASA K. ET AL.: 'Tetsu Howa Shisu 16% Miman no Ketsueki Toseki Kanja ni Okeru Citric Acid Daiichi Tetsu Natrium no Yukosei no Kento' NIPPON TOSEKI IGAKU KAISHI vol. 28, no. 9, 1995, pages 1213 - 1217, XP003025814
- HISANO T. ET AL.: 'Citric Acid Kamususan Tosekieki ni yoru Mususan Ketsueki Toseki (AFHD) no Hyoka' DAI 49 KAI ANNUAL MEETING OF THE JAPANESE SOCIETY FOR DIALYSIS THERAPY YOKOSHU 18 May 2004, XP003025816
- KATAGIRI T. ET AL.: 'Toseki Kanja ni Okeru Citric Acid Naifuku no Rinshoteki Koka ni Tsuite' DAI 48 KAI ANNUAL MEETING OF THE JAPANESE SOCIETY FOR DIALYSIS THERAPY YOKOSHU 20 May 2003, page 997, XP003025815
- BALOGUN R.A.: 'Chronic Kidney Disease: A brief Review for the Primary Care Physician' JCOM vol. 11, no. 12, December 2004, pages 784 - 790, XP008110148
- FRITZ J.C. ET AL.: 'Estimation of the Bioavailability of Iron' JOURNAL OF THE AOAC vol. 58, no. 5, 1975, pages 902 - 905, XP008110147
- TANAKA M. ET AL.: 'Citric Acid Kogyoketsuho ni yoru Ketsueki Toseki' KIDNEY AND DIALYSIS vol. 18, no. 6, 1985, pages 65(789) - 70(794)
- W. H. HÖRL ET AL.: 'Replacement of Renal Function by Dialysis', 2004, KLUWER ACADEMIC PUBLISHERS, DORDRECHT - BOSTON - LONDON pages 387 - 388
- J. T. DAUGIRDAS, M.D. ET AL.: 'Handbook of Dialysis', 1994, LITTLE, BROWN AND COMPANY, BOSTON - NEW YORK - TORONTO - LONDON pages 445 - 468

## Description

### [Technical field]

The present invention discloses iron metabolism-improving agents that improve iron metabolism in the living body, and more specifically, to the iron metabolism-improving agent that improves iron metabolism in a chronic renal failure patient who receives blood purification therapy such as hemodialysis, hemofiltration and hemodiafiltration.

The present invention relates to compositions which constitute dialysate and/or a substitution fluid.

### [Background of the invention]

Chronic renal failure patients who receive blood purification therapy, typically hemodialysis, suffer from lowered quality of life (QOL), hospitalization and high mortality rates induced by various complications. It is well known that causes of such complications include chronic inflammatory state, nutritional deficiencies, arteriosclerosis and erythropoietin (EPO) resistant renal anemia, and that interrelation among them deteriorates prognosis.
Although a comprehensive mechanism causing these pathologies is not known yet, a suspected cause is iron metabolic abnormality induced by renal failure, that is, cellular dysfunction induced by iron accumulation in various organs and tissues as well as increased oxidative stress due to iron-mediated Fenton reactions.

Renal anemia that occurs in chronic renal failure patients receiving blood purification therapy is the pathology of decreased hemopoiesis in the bone marrow resulting from relative underproduction of erythropoietin in renal tissues due to renal dysfunction. A treatment method for renal anemia is administration of recombinant human erythropoietin (rHuEPO) preparations. Recently, rHuEPO preparations are also used for renal failure patients during the conservative period before introduction of dialysis.
However, there are often cases where anemia is not improved despite administration of rHuEPO preparations because of reduced hematopoietic response to EPO-this is EPO-resistant renal anemia, mainly induced by iron deficiency in the bone marrow. It has been considered that, in chronic renal failure patients, increased demand for iron resulting from enhanced hematopoiesis due to rHuEPO treatments causes iron deficiency in the bone marrow. On the other hand, it is also known the state that anemia is improved by additional administration of iron notwithstanding presumable sufficient iron storage considering the serum ferritin concentration-the state of functional iron deficiency.

The present inventors have revealed the existence of iron metabolic abnormality in dialysis patients and a part of its mechanism (Non-patent literature 1). That is, while the serum iron levels of hemodialysis patients were lower than those of healthy subjects, the serum ferritin levels of hemodialysis patients were significantly higher than those of healthy subjects. In addition, the polymorphonuclear leukocyte iron levels of above hemodialysis patients were about 3 times higher and their ferritin levels were about 1.5 times higher than those of healthy subjects.
In evaluation only in patients with lower serum ferritin levels , similar elevations of intracellular iron concentrations were observed.
Hence, the present inventors observed that the polymorphonuclearleukocyte iron levels were enhanced notwithstanding serum ferritin levels in dialysis patients.

Iron transport proteins are considered to relate to intracellular iron metabolism. There have been new findings regarding iron transport proteins at the cellular level, especially in duodenal and reticuloendothelial cells. Well-known proteins that take up iron into cells are transferrin receptor (TfR), which takes up ferric (Fe³⁺)-binding transferrin, divalent metal transporter 1 (DMT1), which transports ferrous (Fe²⁺), and ferroportin 1 (FP1), which exports iron out of cells.

The present inventors have also revealed the relationship between increased intracellular iron concentrations and iron-transport proteins in polymorphonuclear leukocytes (Non-patent literature 1). That is, it was confirmed that TfR relates to the iron uptake into polymorphonuclear leukocytes and FP1 relates to the iron export out of polymorphonuclear leukocytes. In addition, the present inventors have revealed at the mRNA level as well as the protein level that the expression of TfR, an iron-uptake protein, is increased and, contrarily, the expression of FP1, an iron-exporting protein, is decreased in the polymorphonuclear leukocytes of hemodialysis patients compared to healthy subjects.

The above indicates that iron excessively accumulates in polymorphonuclear leukocytes in hemodialysis patients because of the abnormal regulation of above-mentioned iron-transport proteins, that is, an "iron enclosure". Since iron-transport proteins are expressed in the whole-body cells, presumably similar changes of iron-transport protein expression to the changes in polymorphonuclear leukocytes of dialysis patients may occur in the reticuloendothelial system.
The most important iron metabolism in the living body is the regenerating system where iron is recycled for hematopoiesis at the bone marrow by, first, engulfment of senescent erythrocytes in the reticuloendothelial system, followed by extraction of iron from hemoglobin. Under this system, approximately from 20 to 25 mg of iron is recycled per day. When the "iron enclosure" occurs in the hepatic or splenic reticuloendothelial systems or bone marrow macrophages, the regenerating system of iron metabolism is broken down, which leads to the state of functional iron deficiency where iron is deficient notwithstanding high serum ferritin concentration.

Meantime, many chronic renal failure patients are considered to be chronically in the inflammatory state because their levels of C-reactive protein, an indicator of acute inflammatory response, are high. Heretofore, chronic anemia induced by chronic inflammatory diseases have been reported to be caused by iron recycle disorder due to accumulation of iron in splenic and hepatic macrophages as well as due to the "iron enclosure" in the reticuloendothelial system (Non-patent literature 2).
Moreover, a relationship between a part of EPO-resistant renal anemia and chronic inflammatory state has been suggested (Non-patent literature 3).
Recently, given the reports pointing out a relationship between the pathology of anemia induced by chronic inflammatory diseases and inflammatory cytokines (Non-patent literature 4), inflammatory cytokines have received attention as a factor of iron transport protein dysregulation in dialysis patients.

The present inventors have investigated for the influence of inflammatory cytokines on iron transport proteins employing human-monocyte-derived cultured cells (Non-patent literature 5). As a result, increased expression of TfR and DMT1 as well as decreased expression of FP1 were observed at the mRNA and protein levels. In addition, it was observed that similar changes occurred by stimulation of cytokines in the iron-preloaded condition. Hence, itwas indicated that cytokines induce iron transport protein dysregulation in the reticuloendothelial system notwithstanding the intracellular iron concentration.

Since conventional dialysis preparations on the market contain a slight amount (from 8 to 12 mEq/L) of acetic acid for the purpose of stabilization, it has been indicated that acetic acid can promote hypercytokinemia because of its stimulation to monocytes. In the meantime, aconitase, a citric acid-converting enzyme, has iron-sulfur complexes at the active center, and, when the intracellular iron concentration is low, the iron-sulfur complexes decompose to lower the enzyme activity. Also, it has been reported that, among iron-metabolism-controlling proteins (IRPs: iron regulatory proteins) existing in the cytoplasm, IRP1 binds IRE (iron responsive element) existing in the mRNAs of iron transport proteins under the iron deficient condition to inhibit decay of mRNAs of TfR and DMT1 as well as inhibit translation initiation of mRNAs of FP1 (Non-patent literature 6). Because IRP1 is known to activate aconitase when the intracellular iron concentration is high, citric acid can correct the iron transport protein abnormality through reduction of binding between IRP1 and IRE as a result of enhanced aconitase activity.

Summarizing the above, a presumable major cause of EPO-resistant renal anemia is the iron recycle disorder due to "iron enclosure" in the reticuloendothelial system resulting from iron transport protein dysregulation in the reticuloendothelial cultivated cells-like in polymorphonuclear leukocytes-induced by hypercytokinemia developed in dialysis patients. Moreover, this iron recycle disorder can be considered to play a key role in the onset and development of various complications which affect prognosis.

According to the above, it is contemplated that prevention of hypercytokinemia or correction of iron transport protein dysregulation can release "iron enclosure" in the reticuloendothelial system and enhance serum iron concentrations to improve iron recycle in chronic renal failure patients receiving blood purification therapy such as hemodialysis, hemofiltration and hemodiafiltration, and that eventually EPO-resistant renal anemia can be improved and risks of complications can be avoided to improve QOL as well as prognosis of patients.

Heretofore, however, there has been presented no specific method for improvement of iron metabolism abnormality, that is, excessive iron uptake into cells and suppressed iron export out of cells; or for transfer of iron accumulated in the reticuloendothelial system so as to be recycled in the hematopoietic system in chronic renal failure patients receiving blood purification therapy. Conventionally, add-on therapy of iron preparations to EPO preparations has been considered to be effective as a treatment method for chronic renal failure patients with EPO-resistant renal anemia. However, this is one of methods relying on experience of specialized physicians, and therefore, there is no specifically established treatment method at present.

Non-patent literature 1: Otaki Y, et al.: Am. J. Kidney Dis.: 43, 1030-1039 (2004)
Non-patent literature 2: Ali M, et al.: Lancet: 20, 652-655 (1982)
Non-patent literature 3: Barany P: Nephrol. Dial. Tranplant.: 16, 224-227 (2001)
Non-patent literature 4: Ludwiczek S, et al. : Blood. : 101, 4148-4154 (2003)
Non-patent literature 5: Nanami M, et al.: Arterioscler Thromb. Vasc. Biol.: 25, 2495-2501 (2005)
Non-patent literature 6: Hentze MW et al. : Cell: 117, 285-297 (2004)

US 2002/0004530 relates to dialysates used in hemodialysis and renal replacement therapy wherein trisodium citrate is used as a regional anticoagulant. WO2005/094918 (EP 1731183) concerns acetic acid-free solid compositions for preparing dialysates for chronic renal failure ("CRF") patients. Yuasa et al., Nippon Toseki Igakukaishi, 28 (9) 1995, 1213-1217 relates to the use of ferrous citrate as an iron source for dialysis patients. WO 2006/073164 (EP 1834652) concerns a dialysis solution for controlling the decline of ionised calcium in CRF patients. The present invention has as an object to provide iron metabolism-improving compositions that improve iron metabolism in chronic renal failure patients, and more specially, in those who receive blood purification therapy such as hemodialysis, hemofiltration and hemodiafiltration.

The present invention provides compositions which can be used in methods for improving iron metabolism in a chronic renal failure patient who receives blood purification therapy such as hemodialysis, hemofiltration and hemodiafiltration, by administration of iron metabolism-improving compositions provided by the present invention, and more specifically. Thus it makes it possible to provide a method for improving iron metabolism employing a dialysate and/or a substitution fluid.

### [Means for solving the problems]

To solve problems in the conventional art, an embodiment of the present invention comprises a composition
for use in the treatment of iron metabolism abnormality in chronic renal failure patients who are receiving blood purification therapy, said composition being a dialysate composition or substitution fluid containing:
120-150 mEq/L sodium ion
0-5 mEq/L potassium ion
0-5 mEq/L calcium ion
0-2 mEq/L magnesium ion
55-135 mEq/L chloride ion
20-45 mEq/L bicarbonate ion
0.02-5 mEq/L citrate ion and
0-3.0 g/L glucose.
The composition desirably contains no acetic acid or acetate salt.

An embodiment of the invention comprises: the iron metabolism-improving composition as defined in claim 1. The composition of the invention maybe used in a method for improving iron metabolism in a chronic renal failure patient receiving blood purification therapy such as hemodialysis, hemofiltration and hemodiafiltration, by administration of the iron metabolism-improving composition.

### [Advantageous effect of the invention]

The iron metabolism-improving composition provided by the present invention contains citrate ions, and more specifically, contains a citrate salt as an electrolyte; and one of efficacies thereof is improvement of abnormal iron recycle induced by renal failure in a chronic renal failure patient receiving blood purification therapy
Furthermore, the iron metabolism-improving composition provided by the present invention recovers the recycle system of iron metabolism to allow iron to be recycled and eventually prevents hypercytokinemia simultaneously correcting the internal iron transport protein dysregulation in the living body.
Hence, it is one of the excellent advantages of the present invention that improvement of EPO-resistant renal anemia in chronic renal failure patients caused by the iron metabolism-improving composition provided by the invention reduces risks of onset of complications as well as improves QOL and prognosis of patients.

### [Detailed description of the preferred embodiments]

As aforementioned, the iron metabolism-improving composition provided by the present invention contains a citrate salt, and specifically, contains a citrate salt as an electrolyte.
Furthermore the iron metabolism-improving composition provided by the present invention desirably does not contain acetic acid and/or an acetate salt. Instead it contains
a citrate salt as an electrolyte, and additionally, contains other electrolytes and preferably glucose.

Hence, the iron metabolism-improving composition provided by the invention is administered in a formulation of a dialysate or a substitution fluid. A preferable embodiment of such a dialysate or a substitution fluid is a bicarbonate dialysate or a bicarbonate substitution fluid that contains sodium bicarbonate as alkalizer.

Preferable electrolytes used in the iron metabolism-improving agent provided by the present invention in addition to a citrate salt such as sodium citrate include, for example, sodium chloride, potassium chloride, magnesium chloride, calcium chloride, sodium lactate, potassium lactate and calcium lactate. Especially preferable electrolytes include sodium chloride, potassium chloride, calcium chloride, magnesium chloride and sodium citrate.

In addition to above-mentioned components, the iron metabolism-improving agent provided by the present invention preferably contains glucose and pH adjusting agents to suitably control the pH of a dialysate and/or a substitution fluid after preparation.
Preferable pH adjusting agents include citric acid, lactic acid, hydrochloric acid, malic acid, ascorbic acid, tartaric acid and sodium hydroxide. Especially preferable pH adjusting agents include citric acid and hydrochloric acid.

Accordingly, the iron metabolism-improving composition provided by the present invention contains above-mentioned components at the concentrations as described below when suitably diluted and mixed to be a bicarbonate dialysate or a bicarbonate substitution fluid.

| | |
|---|---|
| Sodium ion | 120-150 mEq/L |
| Potassium ion | 0-5 mEq/L |
| Calcium ion | 0-5 mEq/L |
| Magnesium ion | 0-2 mEq/L |
| Chloride ion | 55-135 mEq/L |
| Bicarbonate ion | 20-45 mEq/L |
| Citrate ion | 0.02-5 mEq/L |
| Glucose | 0-3.0 g/L |

When calcium ions are included as an electrolyte in the iron metabolism-improving agent provided by the present invention, insoluble compounds may tend to be produced because of the existence of citric acid and/or a citrate salt such as sodium citrate. However, production of such insoluble compounds is prevented by citric acid with controlling a pH at low level.

According to the present invention, preferably citric acid and/or a citrate salt are contained in the iron metabolism-improving agent in the amount that allows a pH of prepared dialysate to range about between 2.2 and 2.9, and that allows a citrate ion concentration to range between 0.02 and 5 mEq/L as described above.

Moreover, against conventional dialysis preparation "A" and substitution fluid "B" containing acetic acid, the iron metabolism-improving agent provided by the present invention can have an by acetic-acid free formulation. Hence, it is another advantage of the present invention that a dialysate and/or a substitution fluid can be physiologically more compatible because sodium bicarbonate is used as the only alkalizer.

The iron metabolism-improving agent provided by the present invention is capable of correcting iron transport protein dysregulation through reduction of IRP1-IRE binding as a result of enhanced aconitase activity owing to action of citric acid and/or a citrate salt contained therein. In addition, the iron metabolism-improving agent provided by the present invention is also capable of correcting iron transport protein dysregulation through prevention ofhypercytokinemia in dialysis patients owing to acetic-acid free formulation thereof. Consequently, "iron enclosure" in the reticuloendothelial system is released, and then, serum iron concentration increases to improve iron metabolism abnormality, which, as a result, improves iron recycle contributing largely to improvement of EPO-resistant renal anemia.
The above-mentioned points are some of especially unique features of the iron metabolism-improving agent provided by the present invention.

### [Examples]

By way of test and working examples, and not limitation, detailed description of some embodiments of the invention will be set forth.

### Example 1: Effects of dialysis preparations on iron metabolism in renal failure dogs

Renal failure dogs were dialyzed and effects on iron metabolism were evaluated.

### [Experimental method]

After 18-hour fasting, male beagle dogs were anesthetized with pentobarbital sodium (30 mg/kg) via cephalic vein and underwent midline laparotomy followed by complete bilateral ureteral ligation.

Dialysis experiment was performed after 2 days of the operation. Pentobarbital sodium (30 mg/kg) was administered via cephalic vein for introduction. During the operation, pentobarbital sodium was appropriately administered intravenously with an infusion pump to maintain anesthesia.

Blood access was created by an 8 Fr catheter filled with physiological saline mixed with heparin (10 units/mL) implanted into femoral arteriovenous fistula. Then, endotracheal intubation was performed to seta ventilator for respiratory care.

A single-patient hemodiafiltration system, DBG-01 (by Nikkiso Co., Ltd.), was used for hemodialysis. For filter membrane and a blood tubing set, PS-0. 6UW and a pediatric blood tubing set were used, respectively.

For a group, a dialysate provided by the present invention prepared in accordance with the prescription in the table 1 below was used. For the other group, commercial dialysate "AK-Solita™ ·DL" was used. Both groups had three cases, respectively.

Hemodialysis was performed continuously for 4 hours at a blood flow rate of 100 mL/min and at a dialysate flow rate of 300 mL/min without body fluid removal.

For prevention of blood coagulation, 100 units/kg of heparin was administered intravenously in advance of dialysis initiation, and after that, additionally 50 units/kg of heparin was administered intravenously every 1 hour.

During dialysis, pentobarbital sodium was appropriately administered intravenously with an infusion pump to maintain anesthesia.

Blood was collected before bilateral ureteral ligation, before dialysis initiation, and at 2 and 4 hours afterdialysis initiation. Collected blood was centrifuged in a refrigerated centrifuge to measure serum iron, unsaturated iron binding capacity (UIBC), plasma creatinine (Cre), blood urea nitrogen (BUN), citric acid and acetic acid.
Total iron binding capacity (TIBC) was calculated by adding serum iron to UIBC.
After termination of dialysis, blood was removed through blood access with physiological saline, and then after perfusion, splenic tissues were collected from the dissected animals. The collected tissues were stained with prussian blue stain for iron assessment, and the iron stain percentage was computed by image analysis. In addition, immunohistochemical staining of tissue ferritin was performed for macroscopic observation. For tissue analysis, a comparison was performed among two experimental groups consisting of one group administered with the dialysate provided by the present invention and the other group administered with the commercial dialysate, an untreated control group consisting of three healthy cases, and a renal failure control group consisting of three cases untreated with dialysis.

**Table 1.**

| Component | Concentration (mEq/L) | |
|---|---|---|
| | Dialysate Provided by the Invention | Commercial Dialysate |
| Sodium | 140 | 140 |
| Potassium | 2.0 | 2.0 |
| Calcium | 3.0 | 3.0 |
| Magnesium | 1.0 | 1.0 |
| Chlorine | 111 | 111 |
| Glucose | 1.5 | 1 |
| Hydrogencarbonate | 35 | 25 |
| Acetic acid | - | 10 |
| Citric acid | 1.4 | - |
| Sodium citrate | 0.3 | - |

### [Results and Discussion]

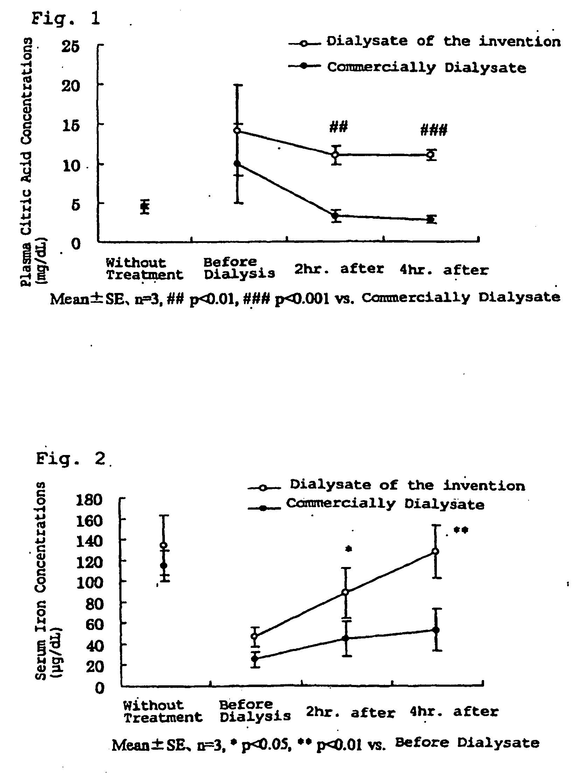

Results of citric acid concentration, serum iron concentration, UIBC and TIBC are shown in Figures 1-4.

The acetic acid concentration results after two hours of dialysis initiation are shown in Table 2.

The plasma Cre and BUN concentrations of dogs in the renal failure group markedly increased after two days of bilateral ureteral ligation before dialysis initiation. The onset of renal failure was confirmed.

The plasma Cre and BUN concentrations having increased due to renal failure decreased immediately after dialysis in both groups administered with the dialysate provided by the invention and the commercial dialysate. While the plasma citric acid concentrations of the group administered with dialysate provided by the invention changed at similar levels to before hemodialysis, the plasma citric acid concentrations of the group administered with the commercial dialysate decreased after dialysis (Figure 1). In comparison between the groups, citric acid concentrations of the group administered with the dialysate provided by the invention were significantly higher than those of the group administered with the commercial dialysate at 2 and 4 hours after dialysis. While plasma acetic acid was not detected in the group administered with the dialysate provided by the invention during dialysis, the plasma acetic acid concentration of the group administered with the commercial dialysis significantly increased due to dialysis (p<0.05, Table 2).

With respect to iron, the serum iron concentrations having decreased due to renal failure significantly increased by dialysis with the dialysate provided by the invention compared to before dialysis (Figure 2). In contrast, no significant change was observed in the group administered with the commercial dialysate. By the two-way analysis of variance, significant differences of iron concentrations were observed not only depending on duration of dialysis session but also on type of dialysates (from before dialysis through 4 h after dialysis: time, F(2,12)=4.46, p<0.05; dialysate, F(1,12)=9.71, p<0.01, time × dialysate, F(2,12)=1.09, p>0.05).

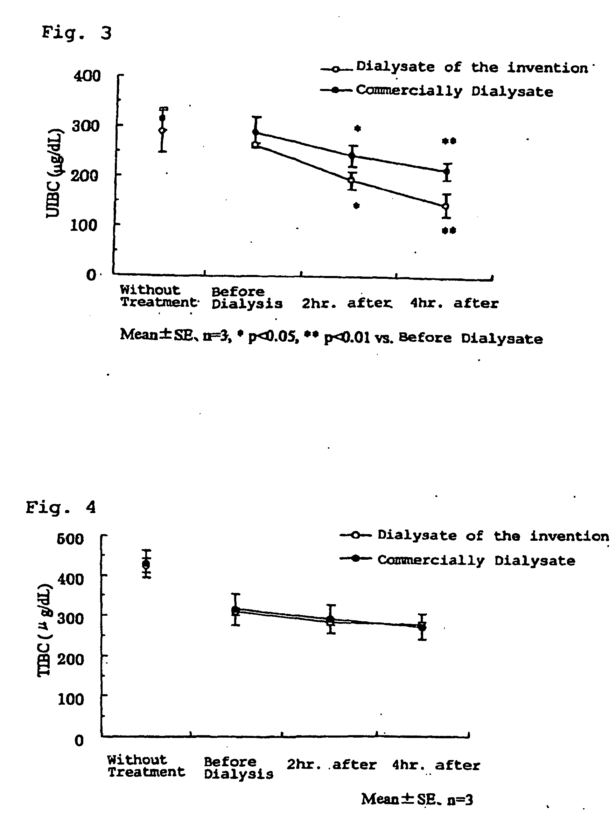

UIBC values after dialysis were significantly lower in both groups administered with the dialysate provided by the invention and the commercial dialysate than before dialysis. By the two-way analysis of variance, significant differences of UIBC values were observed depending on duration of dialysis session as well as on type of dialysates (from before dialysis through 4 h after dialysis: time, F(2,12)=9.91, p<0.01; dialysate, F(1,12)=7.82, p<0.05, time × dialysate, F(2,12)=0.53, p>0.05: Figure 3).

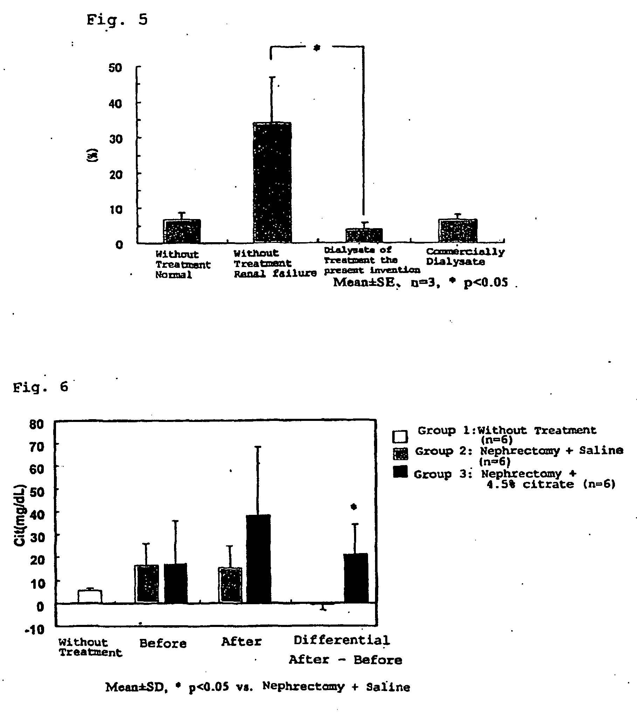

Because practically no change was observed in TIBC values by dialysis and there was no significant difference between the type of dialysates (Figure 4), the changes in UIBC values were considered to be an effect of changes in serum iron concentrations.
The iron stain percentages in splenic tissues of renal failure groups were higher than those of untreated group. The increased iron stain percentages decreased by dialysis; iron staining was less intense in the group administered with the dialysate provided by the invention than iron staining in the group administered with the commercial dialysate (Figure 5). Statistic analysis demonstrated a significant difference in splenic iron stain percentages between the group administered with the dialysate provided by the invention and the group administered with the commercial dialysate (p<0.05). The splenic tissue ferritin staining intensity decreased more, and fewer cells were stained in the group dialyzed with the dialysate provided by the invention than the group dialyzed with the commercial dialysate.
Above analyses of tissues indicate that iron is enclosed in organs under renal failure state and dialysis can release the iron enclosure, and that splenic iron enclosure is markedly released by the dialysate provided by the invention compared to the commercial dialysate.
Accordingly, it is indicated that dialysis with a dialysate provided by the invention can be effective in improvement of declined iron recycle rate due to renal failure.

**Table 2**

| Administration Group | Plasma Acetic Acid Concentration (mg/dL) |
|---|---|
| Dialysate provided by the invention | 0.0 ± 0.0 |
| Commercial dialysate | 4.3 ± 0.2 |
| Note: Examples 2 and 3 do not use compositions embodying the invention but do show the significance of using citrate. | |

### Example 2: Iron metabolism improving effect of citric acid

### administration in bilaterally nephrectomized rats

Effects of citric acid administration on iron metabolism were evaluated in bilaterally nephrectomized rats.

### [Experimental method]

Eight-week-old male CD(SD) rats (body weight: about 300 g) were used in the groups as shown in Table 3. After intra-peritoneal administration of pentobarbital sodium for anesthesia, bilateral total kidneys were extirpated from the back of rat (Rats in the first group in Table 3 were untreated.) and a central venous catheter (CVC) was placed.

At about 24 hours after the operation, 1 mL of blood was collected by CVC. Immediately after blood collection, physiological saline or a 4.5% (w/v) sodium citrate solution was administered by CVC at 0.25 mL/h for 4 hours in the second and third groups in Table 3, and 1 mL of blood was collected by CVC immediately after termination of administration.

Collected blood was centrifuged in a refrigerated centrifuge to measure plasma creatinine (Cre), blood urea nitrogen (BUN), iron and citric acid.

**Table 3**

| Group | Experimental Group | Administered Solution | Number of Animals |
|---|---|---|---|
| 1 | Untreated | - | 3 |
| 2 | Bilateral kidney extirpation + Saline | Physiological saline | 6 |
| 3 | Bilateral kidney extirpation + 4.5% Cit. | 4.5% (w/v) sodium citrate solution | 6 |

### [Results and discussion]

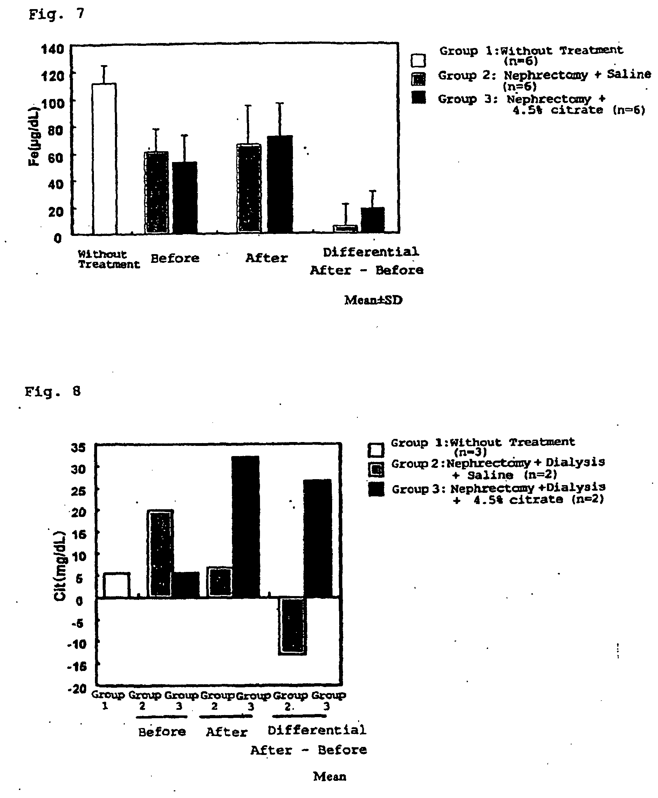

Figure 6 and 7 shows results of citric acid and iron concentrations.

Plasma Cre and BUN concentrations increased markedly at 24 hours after extirpation of bilateral kidneys, and onset of renal failure was confirmed.

Plasma citric acid concentrations were 5.27 ± 1.23 mg/dL in the first group but increased to 16.38 ± 9.49 mg/dL and 17.11 ± 18.64 mg/dL in the second and third groups respectively due to extirpation of bilateral kidneys (Figure 6). The difference of plasma citric acid concentrations between before and after administration in the third group was 21.08 ± 13.18 mg/dL. Enhancement of plasma citric acid concentrations by administration of citric acid was observed. In contrast, the difference of plasma citric acid concentrations between before and after administration was -0.92 ± 2.20 mg/dL in the second group, indicating slight decreases in plasma citric acid concentrations.

Plasma iron concentrations were 112 ± 14 µg/dL in the first group but were 61 ± 17 µg/dL in the second group, indicating decreases due to renal failure state (Figure 7). Onset of iron metabolism abnormality in the second group models was speculated. In the third group administered with citric acid, the difference of plasma iron concentrations between before and after administration was maintained at higher levels (14-18 µg/dL) than that of second group (Δ5 ± 17 µg/dL) (Figure 7). It is indicated that plasma iron concentration can be enhanced by improved iron enclosure due to administration of citric acid.

### Example 3: Iron metabolism improving effect of citric acid administration in a dialysis session in bilateral-kidney-extir-pated rats

Effects of citric acid administration in a dialysis session dialysis on iron metabolism in rat at the second day after extirpation of bilateral kidneys were evaluated.

### [Experimental method]

Examinations were performed in male CD(SD) rats in the groups in Table 4. After intra-peritoneal administration of pentobarbital sodium (50 mg/kg) for anesthesia, bilateral total kidneys were extirpated from the back of rat.

At the second day after operation, after intra-peritoneal administration of pentobarbital sodium (50 mg/kg) for anesthesia, catheters were placed in the left femoral artery and vein to create blood access, and a central venous catheter (CVC) was placed for citric acid administration as well as blood collection. After 1 mL of blood was collected by CVC, dialysis was performed for 4 hours with a commercial dialysate (AK-Solita-DL) under anesthesia. In the second and third groups of Table 4, physiological saline or a 4.5% (w/v) sodium citrate solution was administered by CVC at 1.0 mL/h continuously, and 1 mL of blood was collected by CVC immediately after termination of dialysis.

Collected blood was centrifuged in a refrigerated centrifuge to measure plasma creatinine (Cre), blood urea nitrogen (BUN), iron and citric acid.

**Table 4**

| Group | Experimental Group | Administered Solution | Number of Animals |
|---|---|---|---|
| 1 | Bilateral kidney extirpation | - | 3 |
| 2 | Bilateral kidney extirpation + Dialysis + Saline | Physiological saline | 2 |
| 3 | Bilateral kidney extirpation + Dialysis + 4.5% Cit. | 4.5% (w/v) Sodium citrate solution | 2 |

### [Results and discussion]

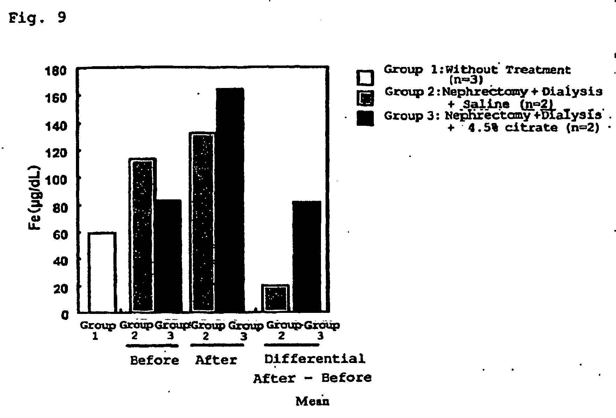

Figures 8 and 9 show results of citric acid and iron concentrations.

Plasma Cre and BUN concentrations increased markedly at the second day after extirpation of bilateral kidneys before dialysis, and onset of renal failure was confirmed. Increased plasma Cre and BUN concentrations decreased immediately by dialysis.

Plasma citric acid concentrations were 5.33 mg/dL in the first group but increased to 9.87.mg/dL in the second group due to extirpation of bilateral kidneys, while plasma citric acid concentrations in the third group were 5.33 mg/dL at the same level as the first group (Figure 8). In the third group administered with citric acid during dialysis, the difference of plasma citric acid concentrations between before and after citric acid administration was 26.49 mg/dL. Enhancement of plasma citric acid concentrations by administration of citric acid was observed. In contrast, in the second group administered with physiological saline during dialysis, the difference of plasma citric acid concentrations between before and after administration was -13.05 mg/dL, indicating slight decreases in plasma citric acid concentrations.

Plasma iron concentrations were 59 µg/dL in the first group, 113 µg/dL in the second group and 83 µg/dL in the third group. Although there were slight differences among the groups, decreases of plasma iron concentrations due to renal failure state induced by extirpation of bilateral kidneys were observed (Figure 9). In the third group administered with citric acid during dialysis, the difference of plasma iron concentrations between before and after administration was maintained at about 4 times higher (81 µg/dL) than that of second group (Δ19.5 µg/dL) (Figure 9). It is indicated that plasma iron concentration can be enhanced by improvement in iron enclosure due to administration of citric acid.

### [Industrial applicability]

As aforementioned, according to the present invention, an acetic acid- and/or acetate salt-free iron metabolism improving agent containing citric acid and/or a citrate salt prevents hypercytokinemia, corrects iron transport protein dysregulation and releases "iron enclosure" in the reticuloendothelial system, which leads to enhancement of serum iron concentrations and improvement in iron recycle in a chronic renal failure patient receiving blood purification therapy. As a result, EPO-resistant renal anemia of such a patient is improved, and complication occurrence risks are reduced. Hence, the present invention has great medical advantages and usefulness for its contribution to improvement of QOL and prognosis of patients.

### [Brief description of the drawings]

Figure 1 shows the results of plasma citric acid concentrations in Example 1.
Figure 2 shows the results of serum iron concentrations in Example 1.
Figure 3 shows the results of UIBC in Example 1. Figure 4 shows the results of TIBC in Example 1.
Figure 5 shows the results of iron stein of splenic tissues.
Figure 6 shows the results of plasma citric acid concentrations in Example 2.
Figure 7 shows the results of plasma iron concentrations in Example 2.
Figure 8 shows the results of plasma citric acid concentrations in Example 3.
Figure 9 shows the results of plasma iron concentrations in Example 3.

## Claims

1. Composition for use in the treatment of iron metabolism abnormality in chronic renal failure patients who are receiving blood purification therapy, said composition being a dialysate composition or substitution fluid containing:
120-150 mEq/L sodium ion
0-5 mEq/L potassium ion
0-5 mEq/L calcium ion
0-2 mEq/L magnesium ion
55-135 mEq/L chloride ion
20-45 mEq/L bicarbonate ion
0.02-5 mEq/L citrate ion and
0-3.0 g/L glucose.

2. A composition according to claim 1 which is a dialysate composition.

3. A composition according to claim 1 which is a substitution fluid.

4. A composition according to claim 1, claim 2 or claim 3 which is free from acetic acid and acetates.

## Patentansprüche

1. Zusammensetzung zur Verwendung bei der Behandlung einer Anomalie des Eisenstoffwechsels bei Patienten mit chronischem Nierenversagen, die eine Blutreinigungstherapie erhalten, wobei die Zusammensetzung eine Dialysatzusammensetzung oder ein Substitutionsfluid ist, die/das Folgendes enthält:
120-150 mÄquiv./l Natriumion
0-5 mÄquiv./l Kaliumion
0-5 mÄquiv./l Calciumion
0-2 mÄquiv./l Magnesiumion
55-135 mÄquiv./l Chloridion
20-45 mÄquiv./l Bicarbonation
0,02-5 mÄquiv./l Citration und
0-3,0 g/l Glucose.

2. Zusammensetzung nach Anspruch 1, die eine Dialysatzusammensetzung ist.

3. Zusammensetzung nach Anspruch 1, die ein Substitutionsfluid ist.

4. Zusammensetzung nach Anspruch 1, Anspruch 2 oder Anspruch 3, die frei von Essigsäure und Acetaten ist.

## Revendications

1. Composition pour utilisation dans le traitement d'une anomalie du métabolisme du fer chez des patients présentant une insuffisance rénale chronique, qui reçoivent une thérapie de purification du sang, ladite composition étant une composition de dialysat ou fluide de substitution contenant:
120-150 mEq/L ions de sodium
0-5 mEq/L ions de potassium
0-5 mEq/L ions de calcium
0-2 mEq/L ions de magnésium
55-135 mEq/L ions de chlore
20-45 mEq/L ions de biocarbonate
0,02-5 mEq/L ions de citrate et
0-3,0 g/L du glucose

2. Composition selon la revendication 1, qui est ladite composition de dialysat.

3. Composition selon la revendication 1, qui est un fluide de substitution.

4. Composition selon la revendication 1, la revendication 2 ou la revendication 3, qui est exempte d'acide acétique et d'acétates.
